Europäisches Patentamt

European Patent Office

Office européen des brevets

(11) Publication number: **0 503 208 A1**

## EUROPEAN PATENT APPLICATION

(21) Application number: **91500025.1**

(22) Date of filing: **08.03.91**

(51) Int. Cl.⁵: **A61K 35/78**

(43) Date of publication of application:
**16.09.92 Bulletin  92/38**

(84) Designated Contracting States:
**AT BE CH DE DK ES FR GB GR IT LI LU NL SE**

(71) Applicant: **MARACUYAMA INTERNATIONAL, S.A.**
**Via Brasil, 26, Apartado 5.289**
**Panama 5(PA)**

(72) Inventor: **Delgado de Chang, Leyda**
**Apartado 3286, Zona 4**
**Panama(PA)**

(74) Representative: **Cobas Barrios, Luis**
**c/o Tecnieurop, S.L., Salustiano Olozaga, 6**
**E-28001 Madrid(ES)**

(54) **Procedure for obtaining a natural water-soluble extract from the leaves and/or rhizomes of various immunologically active ferns.**

(57) This invention concerns a procedure for obtaining a natural water-soluble extract from the leaves and/or rhizomes of various immunologically active ferns.

EP 0 503 208 A1

## SUMMARY OF THE INVENTION

This invention concerns a procedure for obtaining a natural water-soluble extract from the leaves and/or rhizomes of various immunologically active ferns.

## BACKGROUND TO THE INVENTION

The human immune system consists basically of the phagocyte, humoral and cellular systems. Though each has different functions, in fact they act interdependently so that a deficiency or depression of one of them causes functional abnormalities in the others.

In the humoral immune system (B lymphocytes) we find the energetic and resolute response by means of antibodies to antigenic aggression from pathogenic bacteria and germs.

In the cellular immune system (T lymphocytes) we have the regulating agents for the immune response and defence against infection by intracellular germs (viruses) and mutant cells.

From among the T lymphocytes we can distinguish: Th lymphocytes or collaborators which have a dual regulatory action in the positive sense, on the one hand acting on B cells, stimulating their differentiation into plasmatic cells which have to secrete antibodies and, on the other hand, positively regulating the precursors of the cytotoxic T cells.

Tk or cytotoxic lymphocytes which play a role in the response to and destruction of cells infected by a virus and are the causal agents of immune reactions at the cellular level, such as the rejection of grafted and neoplasia cells. These lymphocytes are capable of carrying out the most effective lysis of cells infected by a virus, irrespective of the virus invading them. This makes these cells the most potent antiviral agents known.

Finally, the Ts or suppressor lymphocytes, unique in being capable of negatively regulating differentiation of B lymphocytes and preventing the positive activity of Th cells, which would aid the development of B cells and lead to a collapse in the antibody secretory function of plasmatic cells.

Ts lymphocytes are therefore capable of suppressing the immune response of B cells, a particularly important phenomenon this in situations where it is necessary to moderate, regulate or suppress an antibody response to an autoantigen.

When there is a functional deficiency of Ts lymphocytes, an autoantibody/autoantigen confrontation can appear, which will give rise to an autoimmune pathology, always self-aggressive and in many cases with little truly effective therapeutic storehouse.

A correctly functioning cellular immune system is the most effective defence against viral infections. It can be decisive in the prevention and cure of neoplasia, as well as playing a role in the normal development of the homeostatic process. Its dysfunctionalism can also lead to the appearance of a wide range of autoimmune-type chronic ailments.

Among the illnesses that occur or may occur with a deficiency of T suppressor lymphocytes, the following can be noted:

Rheumatoid arthritis, erythematosus lupus, Sjöengren's syndrome, type B active chronic hepatitis, Di George's syndrome, thrombocytaenia, autohaemolitic anaemia, atopic dermatitis, psoriasis, Basedow's disease, Crohn's disease, myasthenia gravis, ataxic telangiectasia, vitiligo, area celsi, herpes zoster, etc.

## OUTLINE OF THE INVENTION

It has been discovered that the natural water-soluble extract isolated from the rhizomes and leaves of Phlebodium decumanum, Polypodium aureum, Polypodium leucotomos, Polypodium vulgare, Polypodium triseriale, Pteridium aquilinum, Dryopteris crassirhizoma, and Cyathea taiwaniana exercise a specific and definitive action at the level of the cellular immune system. It significantly increases the percentage of T suppressor lymphocytes without altering the Th:Ts ratio, without producing leukopenia, and without altering the normal seric levels of immunoglobulin.

When administered to patients with pathologies in which a depression of the cellular immune system is implicated - generally due to a deficiency of T suppressor lymphocytes (autoimmune illnesses and herpetic infections) - this water-soluble extract has demonstrated that it possesses a highly positive therapeutic action in their treatment.

In double-blind studies carried out on 45 patients affected by herpes zoster, the treatment showed that it could cut the infection's natural evolution time to half without any of the typical post-herpetic neuralgias appearing in 68 percent of patients. Similar results were obtained in two open studies carried out on 85 patients also affected by herpes zoster.

2

The average treatment time was 21 days, with a dose of 10 mg/kg weight per day.

In controlled monitoring over four years on patients affected with multiple sclerosis, with very varied degrees of evolution, it was proven that, after starting treatment with this substance, the recurring outbreaks typical of this illness after apparent remissions disappeared completely. It was even possible to note some functional recoveries. When the medication was withdrawn or the dose reduced to less than 720 mg a day, the illness regained its natural evolution and the outbreaks reappeared, even in patients who had received more than three years' continual treatment.

For atopic dermatitis, in controlled studies on 253 patients of various ages, the disappearance of the pruritus was noticed and a notable improvement or disappearance of lesions in 90 percent of patients treated. The disappearance of relapses was clearly evident.

In ophthalmologic pathology, controlled studies on 55 patients produced clinical results that were highly positive at four levels: epithelamic, with regeneration of the corneal epithelium; stromatic, (corneal ulcers, leucomas); endothelic (striae in Descemet's membrane); and immunological (Sjöegren's and Behçet's syndromes).

Another study on 27 patients affected with various viral keratoses, showed particularly strongly the absence of relapses, as well as complete effectiveness against the viral infection.

In these kinds of viral or autoimmune infections, the normal daily dose varied between 15 and 20 mg/kg weight per day.

From the toxicological point of view, no effect has been discovered. During 18 years' research, involving haematological and bone marrow examinations, tests on renal and hepatic functioning, and urine analysis, there was no sign of any change whatsoever. Following standards set down in the FRG and USA, and in accordance with "Guidelines for Reproduction Studies for Safety Evaluation of Drugs for Human Use", these compounds have no effect on fertility, neither in mice and rats nor in mammals (dogs). Nor has any teratogenic effect been observed in four generations of rats and mice and two generations of dogs.

Nor has any carcinogenic activity been observed. These studies were carried out on mice, which were administered a daily dose of one milligram per kilogram body weight via the oral route for as period of 24 weeks. The animals were then killed starting from seven weeks after that, with and without glass-bead implantation in the urinary bladder. The methods used in these studies are described by Jull; J.W. Brit J. Cancer 5 328 (1951). The statistical evaluation process used is described in Arcos et al. Chemical Induction of Cancer, page. Academia Press Inc. New York, (1963).

The possible action mechanism of the water-soluble extract could be that described in the following diagram:

The increase in T suppressor lymphocytes signifies:

a) Reestablishment of the cellular immune system's functionality, which had been depressed by the deficiency of Ts lymphocytes.

b) Recovery of antiviral activity among cytotoxic lymphocytes.

c) Recovery among Ts lymphocytes of their response capacity to autoimmune reaction, measured by autoantibodies.

d) Correct functioning of the T lymphocytes in the synthesis and release of low molecular weight proteins, such as lymphokenes and various soluble factors that play a decisive role in the healing process and in the stimulation of macrophages and fibroblasts that are to synthesize the precollagen, etc.

The water-soluble extract does not have any steroid action.

Regarding its effect on other organs and systems, it has a bradycardial effect similar to that of digitalins, possibly due to its action on myocardial cellular membranes.

EXTRACTION PROCEDURE

The ferns used are as follows: Polypodium aureum, Polypodium leucotomos, Polypodium vulgare, Polypodium triseriale, Dryopteris crassirhizoma, Cyathea taiwaniana and Pteridium aquilinum.

The method that is the subject of this invention includes the following stages:

COLLECTING THE PLANT. Cutting the plant and its collection for the purpose of obtaining the maximum yields will be carried out with reference to the plant having developed young sporangia.

DRYING. The rhizomes are chopped up in a hammer grinder before they are dried in order to facilitate this process.

The drying is carried out continuously in a belt dryer, using an air current at between 65°C and 70°C. The raw material on the belt in left to dry for about two hours.

The residual humidity of the raw material obtained is less than 7 percent.

MILLING. The dry product is milled in a disc mill in order to obtain particles of size less than 3 mm.

EXTRACTION-EVAPORATION: The extraction stage consists of three phases:

1) Extraction with petroleum ether. The solution is recovered and the remaining solid matter is discarded.

2) Extraction with solvents of dielectric constant between 1.8 and 9.5 (hexane, dichloromethane, etc.). We carry out this extraction with a hexane/dichloromethane mixture (1:1 by volume). We evaporate the solution to obtain a final residual volume equivalent to 1/10 of the initial volume. We call this Fraction A.

3) Extraction with polar solvents. In this phase we use solvents with a higher dielectric constant (30-85). We carry out the extraction with a methanol/water mixture (60:40 by volume). Varying the proportion varies the yield obtained. We then evaporate to obtain a residual volume of 1/10 the initial volume. We call this Fraction B.

PURIFICATION. Fractions A and B are mixed together and an equal volume of methanol/water mixture (1:1) is added to it. The whole is agitated vigorously for 30 minutes in order to mix the phases. They are then left to separate out in decantation vessels for a period of 15 hours. The organic phases and separated and discarded.

The separated aqueous solution is filtered and passed through acid-base ion exchanger resins.

The solution obtained is neutralized with calcium hydroxide.

After neutralization, the extract is clarified by the addition of activated charcoal. After agitating for 15 minutes, the activated charcoal is removed by filtration through a filtering cartridge and a transparent colourless solution is obtained.

The solution obtained from filtering is vacuum concentrated at a temperature no greater than 55°C until the volume is reduced by half. The concentrated extract obtained is precipitated out by the addition of an equal volume of a water-miscible organic liquid, such as absolute alcohol.

The precipitate is separated by vacuum filtration and discarded, the parent solution being retained and vacuum concentrated at a temperature no greater than 55°C until a yellow-brown product with a humidity between 25 and 30 percent is obtained. This product has the appearance and consistency of honey and is the subject of this invention.

As can be seen from the procedure for obtaining the extract, the product is not a pure chemical with a definite composition. Rather, it has a composition determined by the nature of the plant used and by the extraction and purification methods.

The presence of sugars can be detected in the extract, particularly D-glucose and D-fructose, confirmed by the usual methods. Also acids can be detected difficult to separate from the sugars. Their presence can be confirmed by chromatographic and spectroscopic methods.

The aim of this invention is to establish the pharmaceutical preparations resulting from the combination of the water-soluble extract with suitable pharmaceutical vehicles in order to make its administration in human pathological processes useful and feasible.

As an active substance, the water-soluble extract will be the basic ingredient of this invention's compositions, being incorporated in appropriate amounts to produce immunological activity.

The pharmaceutical compositions resulting from this invention contain the active substance in unitary doses of 40 mg, 120 mg and 240 mg, with the minimum active dose being 80 mg and the maximum 1200 mg. This is in order to compensate for different among the age and weight of patients and also for the treatment process, which may be acute and of short duration or chronic and of long duration.

Due to its virtual lack of toxicity, its tolerance, its lack of secondary effects and its good gastric-duodenal absorption, the preferred administration route is oral. Administration of the active substance, varying as described above, is between 80 mg and 1200 mg in 2-3 daily preprandial doses, divided as equally as possible through the day so that concentrations in the blood are kept at a stable and continual immunologically active level.

The extract that is the subject of this invention can be used in various pharmaceutical forms for dosing, such as:

Pills, capsules: For this the product is granulated with suitable excipients and prepared for the later production of unitary doses in capsule or pill form. Lactose, starch, talc, Mg stearate, aerosil and similar compounds are the usual excipients in this preparation.

Syrups: In this case the product is prepared with liquid vehicles such as alcohols (ethanol, propylene-glycol, glycerin, etc.), oils (peanut, olive, sunflower, etc.) and other solubilizers, preservatives and aromatizers.

Injection: For parenteral use, the extract is offered in sterilized phials and/or ampoules with liquid vehicles such as water, alcohols and preservatives.

As illustrative data of the process, we could describe an example.

1 kg of fern is extracted with 10 litres of solvent in each of three extractions. The average yield obtained in 10 tests is 22.3 g of extract.

Below we describe some examples of pharmaceutical preparations in capsules:

## EXAMPLE 1

| Raw material | Quantities per capsule | Quantities per 100,000 cap. batch |
|---|---|---|
| Water-soluble extract | 120 mg | 12 kg |
| Starch | 180 mg | 18 kg |
| Lactose | 180 mg | 18 kg |
| Mg stearate | 15 mg | 1.5 kg |
| Aerosil 200 | 1 mg | 0.1 kg |

The extract, the starch and the lactose are placed in a mixer and blended for half an hour. The mixture is then granulated and vacuum dried until a mixture with humidity less than 3 percent is obtained. This is then milled and sieved through a No. 30 screen.

The product obtained after sieving is mixed with the magnesium stearate and the Aerosil 200. Next, this mixture is dosed into hard gelatine capsules at the rate of 496 mg pr capsule - i.e., 120 mg of the active principle.

## EXAMPLE 2

| Raw material | Quantities per capsule | Quantities per 100,000 cap. batch |
|---|---|---|
| Water-soluble extract | 120 mg | 12 kg |
| Starch | 100 mg | 10 kg |
| Lactose | 150 mg | 15 kg |
| Talc | 115 mg | 11.5 kg |
| Mg stearate | 10 mg | 1 kg |
| Aerosil 200 | 1 mg | 0.1 kg |

We proceed in the same way as in example 1. First the water-soluble extract, the starch and the lactose are mixed together and granulated, dried, milled and sieved. They are then mixed with the remaining excipients and dosed at 496 mg/capsule.

## Claims

1. Procedure for extracting a natural water-soluble extract with immunological activity starting from the rhizomes and/or leaves of: Phlebodium decumanum, Polypodium aureum, Polypodium leucotomos, Polypodium vulgare, Polypodium triseriale, Dryopteris crassirhizoma, Cyathea taiwaniana and Pteridium aquilinum, characterised by consisting of the stages: collecting the plants, taking as a reference the fact that they have developed young sporangia; drying said plants in a belt dryer with hot air at a temperature between 65°C and 70°C until a residual humidity less than 7 percent is obtained; milling the resulting product in order to obtain particles smaller than 3 mm; extracting the milled product in three phases; concentrating the extracts; and submitting the concentrate to later liquid-liquid extraction, passing through ion exchanger resins, neutralization, clarification with activated charcoal, precipitation with alcohol and obtaining the parent liquid, which is vacuum concentrated at a temperature less than 55°C to obtain the required extract.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | GB-A-2024622 (CONRAD LIMITED) <br> * page 1, lines 35 - 44 * <br> * page 2, lines 8 - 45 * <br> --- | 1 | A61K35/78 |
| Y | GB-A-2022094 (CONRAD LIMITED) <br> * page 1, column 2, line 116 - page 2, column 2, line 67 * <br> --- | 1 | |
| Y | DERWENT PUBLICATIONS Ltd, LONDON, GB; <br> DATABASE WPIL <br> accession no. 90-141716, week 9019; <br> & ES-2012734 [VARGAS GONZALEZ J.F.],1 april 1990 <br> --- | 1 | |
| Y | FR-A-2479690 (CONRAD LIMITED) <br> * pages 3 - 5 * <br> --- | 1 | |
| Y | US-A-3395223 (MC CAN BERGER F. ET AL) <br> * claims 1-9 * <br> --- | 1 | |
| Y | US-A-4886666 (YAGUANG LIU) <br> * column 3, line 50 - column 4, line 8 * <br> --- | 1 | TECHNICAL FIELDS SEARCHED (Int. Cl.5 ) |
| A | BE-A-706040 (MANUFACTURE DE PRODUITS PHARMACEUTIQUES A. CHRISTIAENS) <br> * page 1 * <br> --- | 1 | A61K |
| A | US-A-3839553 (MARTINEZ J.M. ET AL) <br> * column 2, line 25 - column 3, line 10 * <br> --- | 1 | |
| A | GB-A-2000765 (SOCIETE CIVILE PARTICULIERE DE BREVETS SUFFREN) <br> * page 1, column 2, line 120 - page 2, column 1, line 51 * <br> ----- | 1 | |

The present search report has been drawn up for all claims

| Place of search | Date of completion of the search | Examiner |
|---|---|---|
| BERLIN | 16 OCTOBER 1991 | AVEDIKIAN P.F. |

EPO FORM 1503 03.82 (P0401)